# FASCICULE DE BREVET EUROPEEN

(11) **EP 1 769 787 B1**
(45) Date de publication et mention de la délivrance du brevet: **22.08.2012**
(21) Numéro de dépôt: 06301006.0
(22) Date de dépôt: 03.10.2006
(51) Int. Cl.: A61K 8/11, A61K 8/25, A61K 8/04, A61Q 1/02, A61Q 3/02

(54) **Composition cosmétique fluorescente**
Fluoreszierende kosmetische Zusammensetzung
Cosmetic fluorescent composition

(30) Priorité: 03.10.2005 US 722448 P
(43) Date de publication de la demande: 04.04.2007
(73) Titulaire: L'Oréal, 75008 Paris (FR)
(72) Inventeur: Dumousseaux, Christophe, Tokyo (JP)
(74) Mandataire: Tanty, François

(56) Documents cités:
- WO-A-2005/009604
- WO-A-2005/011622
- WO-A2-2004/081222

## Description

La présente invention concerne une composition cosmétique destinée à être appliquée sur les matières kératiniques, notamment la peau, les cheveux, les lèvres, les ongles ou les cils.

Les pigments ou les colorants fluorescents présentent un grand intérêt en cosmétique. Ils permettent d'obtenir des couleurs très brillantes et très vives qui ne sont pas atteignables avec des matières colorantes classiques. Lorsqu'ils n'absorbent pas dans le visible ils permettent également d'éclaircir et d'augmenter significativement la réflectance des substrats sur lesquelles ils sont déposés comme la peau et le cheveu.

Parmi les différents matériaux fluorescents disponibles, il est connu de l'homme de l'art que ce sont les molécules fluorescentes organiques qui présentent la plus grande efficacité en terme d'intensité de fluorescence. Leur utilisation permet d'obtenir des compositions très fluorescentes et contenant une faible quantité d'agent fluorescent.

Ces molécules organiques sont cependant difficile à formuler car elles sont en général solubles dans un nombre très limité de solvants. Leur intensité de fluorescence dépendra également fortement du solvant utilisé dans la formulation et elles ne seront fortement fluorescentes que dans un faible nombre de solvants. Ceci limite donc leur possibilité d'utilisation dans des formulations cosmétiques. Elles peuvent également présenter un problème de sécurité lorsqu'elles sont en contact avec les matières kératiniques. Afin de pallier ces inconvénients il a été envisagé d'encapsuler des molécules organiques dans différentes matrices organiques (US 6 586 013) ou inorganiques (JP06-47273 et JP08-239310). Cependant toutes les matrices utilisées jusqu'à maintenant présentent une certaine porosité et laissent diffuser la molécule à l'extérieur. Cette molécule peut donc se retrouver dans le milieu continu liquide de la formulation ce qui peut entraîner un contact avec les matières kératiniques.

### Résumé

L'invention a pour objet une composition cosmétique contenant dans un milieu physiologiquement acceptable, des particules fluorescentes comprenant des molécules d'au moins un composé organique fluorescent encapsulées à l'intérieur d'une matrice formée au moins en partie d'au moins un oxyde métallique et de phosphore.

Les particules ainsi obtenues, lorsqu'elles sont introduites dans une composition cosmétique, permettent de conférer des propriétés de fluorescence à cette dernière.

Avantageusement, la matrice comprend en outre une faible proportion de groupements organiques, tels que des groupements phényls, alkyls. La fonction de ces groupements organiques est de créer une interaction, notamment via les forces de Van der Waals ou les forces par liaison hydrogène, avec les molécules organiques fluorescentes de manière à en limiter le dégorgement dans le milieu de formulation.

Selon l'invention, le dégorgement des molécules organiques peut être limité en augmentant la densité du réseau d'oxydes métalliques, en ajoutant à ces derniers une faible proportion de phosphore sous forme d'acide ou d'ester phosphorique, apte à créer avec les oxydes métalliques des ponts bi ou tri fonctionnels.

Les molécules organiques fluorescentes peuvent être de différentes natures chimiques et peuvent être colorées ou non colorées. On peut citer notamment les molécules de la famille des fluorescéine, des pyrazines, des coumarines, des naphtalimides, des triazines, des dioxazines, des sulforhodamines, des azoiques, des azomethiniques, les dérivés du stilbene, les dérivés oxazole, benzoxazole, imidazole.

Les molécules fluorescentes peuvent être hydrosolubles, liposolubles ou solubles dans d'autres solvants comme les alcools.

Les molécules fluorescentes de l'invention sont de préférence complètement ou partiellement hydrosolubles, c'est-à-dire, solubles dans l'eau pour une concentration d'au moins 1 mg/L, de préférence au moins 10 mg/L.

Comme molécule non colorée on pourra utiliser les azurants optiques absorbant la lumière entre 300 et 400nm et émettant la lumière entre 400 et 700nm, de préférence entre 400 et 500nm. De tels azurants optiques sont disponibles commercialement auprès de CIBA SPECIALTY CHEMICALS dans la gamme Uvitex® ou Tinopal®, auprès de CLARIANT dans la gamme Leucophor®, auprès de BAYER CHEMICAL dans la gamme Blankophor® ou auprès de BASF dans la gamme Ultraphor®.

Comme molécule colorée, on utilisera de préférence les molécules suivantes listés par la FDA : D&C Yellow 7, 8, 10, 11, D&C Red 3, 21, 22, 27, 28, D&C Green 5, 6, 8, D&C Orange 5, D&C Violet 2, ext D&C Violet 2.

Selon un mode de réalisation particulier, la matrice est réalisée à l'aide d'une synthèse sol-gel à partir d'alcoxyde métallique en présence d'acide, d'ester ou de sel phosphorique, suivi d'un séchage à une température entre 80 et 200 °C. En pratique on peut utiliser de l'acide phosphorique, du phosphate de calcium, de magnésium ou de sodium. L'acide phosphorique H3PO4 est utilisé préférentiellement. Sa présence permet de forme un réseau très dense lors de la condensation de l'oxyde métallique, notamment grâce à la formation de ponts phosphates. Sa concentration pourra être comprise entre 0.01% et 0.25% par rapport au poids total de la matrice. L'alcoxyde métallique est de préférence choisi parmi les alcoxydes de silicium, de titane, d'aluminium ou de zirconium. De façon à obtenir une matrice transparente on utilisera de préférence un alcoxyde de silicium. Cet alcoxyde de silicium contient au moins un groupement Si(OR), avec R groupement organique qui est de préférence une chaine alkyle contenant 1 à 5 atomes de carbone.

La matrice inorganique des particules utilisées dans les compositions selon l'invention est non soluble dans le milieu continu de la formulation. Les particules sont ainsi insensibles aux solvants et aux conditions de température.

L'absorption d'huile des particules utilisées dans les compositions selon l'invention est de préférence très faible. Typiquement, elle est comprise entre 10 et 100 ml/100g, de préférence entre 20 et 60ml/100g.

La concentration en molécules fluorescentes dans les particules peut être comprise de 0.01 à 50% en poids, de préférence de 0.1 a 20% en poids, de préférence entre 0.5 et 5%.

Les particules peuvent contenir un mélange de plusieurs molécules organiques fluorescentes. Elles peuvent également contenir d'autres composés, fluorescents ou non, (nanoparticules inorganiques fluorescentes ou nanoparticules d'oxydes métalliques par exemple).

Les particules peuvent être présentes dans les compositions selon l'invention à une concentration comprise entre 0.5 et 95%, de préférence entre 1 et 70%, de préférence entre 5 et 30%.

### Composés additionnels

Les compositions selon l'invention peuvent contenir un ou plusieurs composés additionnels choisis notamment parmi ceux décrits ci-après.

### Elastomère de silicone polyglycérolé

La composition selon l'invention peut notamment contenir un élastomère de silicone polyglycérolé.

L'élastomère de silicone polyglycérolé présent dans la composition peut être un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

De préférence, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité de 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyl, éthyl, propyl, butyl, octyl, décyl, dodécyl, (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, wylyl ; des groupes aryles substitués tels qu'un groupe époxy, un groupe ester caboxylate, ou un groupe mercapto. De préférence, ledit groupe organique est choisi parmi les groupes méthyl, phényl, lauryl.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane- méthylhydrogénosiloxane-laurylméthylsiloxane à terminaisons triméthylsiloxy.

Le composé (B) peut être un composé polyglycérolé répondant à la formule (B') suivante :

CₘH₂ₘ₋₁ -O-[Gly]ₙ-CₘH₂ₘ₋₁ (B')

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :

-CH₂-CH(OH)-CH₂-O- ou -CH₂-CH(CH₂OH)-O-

La somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation, et est notamment l'acide chloroplatinique, les complexes acide chloroplatinique-oléfme, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 100 parts en poids de la quantité totale des composés (A) et (B).

L'élastomère de silicone polyglycérolé peut être véhiculé sous forme de gel dans au moins une huile hydrocarbonée et/ou une huile siliconée. Dans ces gels, l'élastomère polyglycérolé est souvent des particules non-sphériques.

Comme élastomère de silicone polyglycérolés, on peut utiliser ceux vendus sous les dénominations « KSG-710 », « KSG-810 », « KSG-820 », « KSG-830 », « KSG-840 » par la société SHIN ETSU.

L'élastomère de silicone polyglycérolé peut être présent dans la composition en une teneur allant de 0,1 % à 50 % en poids, par rapport au poids total de la composition, de préférence allant de 0,1 % à 40 % en poids, préférentiellement allant de 0,5 % à 30 % en poids, plus préférentiellement allant de 0,5 % à 20 % en poids, et encore plus préférentiellement allant de 1 % à 10 % en poids.

### Elastomère de silicone émulsionnant

La composition selon l'invention peut comprendre un élastomère de silicone émulsionnant différent de l'élastomère de silicone polyglycérolé.

Par élastomère de silicone émulsionnant on entend un élastomère de silicone comprenant au moins une chaîne hydrophile différente d'une chaîne polyglycérolée telle que décrite précédemment.

En particulier, l'élastomère de silicone émulsionnant additionnel peut être choisi parmi les élastomères de silicone polyoxyalkylénés.

L'élastomère de silicone polyoxyalkyléné est un organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'organopolysiloxane réticulé polyoxyalkyléné est obtenu par réaction d'addition réticulation (A1) de diorganopolysiloxane contenant au moins deux hydrogènes liés chacun à un silicium, et (B1) de polyoxyalkyléné ayant au moins deux groupements à insaturation éthylénique, notamment en présence (C1) de catalyseur platine, comme par exemple décrit dans les brevets US 5 236 986 et US 5 412 004.

En particulier, l'organopolysiloxane peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfme, les complexes acide chloroplatinique-alkenylsiloxane, les complexes acide chloroplatinique-dicétone, le platine noir, et le platine sur support.

Les élastomères de silicone polyoxyalkylénés peuvent être formés à partir de composés divinyliques, en particulier des polyoxyalkylènes ayant au moins deux groupes vinyliques, réagissant avec des liaisons Si-H d'un polysiloxane.

Les élastomères de silicone polyoxyalkylénés peuvent être véhiculés sous forme de gel constitué d'un organopolysiloxane élastomérique inclus dans au moins une huile hydrocarbonée et/ou une huile siliconée. Dans ces gels, les particules d'organopolysiloxanes sont souvent des particules non-sphériques.

Des élastomères polyoxyalkylénés sont notamment décrits dans les brevets US 5 236 986, US 5 412 004, US 5 837 793, US 5 811 487.

Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-310", "KSG-320", "KSG-330", "KSG-340", "X-226146" par la société SHIN ETSU, "DC9010", "DC9011" par la société DOW CORNING.

L'élastomère de silicone émulsionnant additionnel peut être présent dans la composition en une teneur allant de 0,1 % à 50 % en poids, par rapport au poids total de la composition, de préférence allant de 0,1 % à 40 % en poids, préférentiellement allant de 0,5 % à 30 % en poids, plus préférentiellement allant de 0,5 % à 20 % en poids, et encore plus préférentiellement allant de 1 % à 10 % en poids.

### Elastomère sphérique non émulsionnant

La composition selon l'invention peut comprendre un élastomère sphérique non émulsionnant.

Le terme "non émulsionnant" définit des élastomères organopolysiloxane ne contenant pas de chaîne hydrophile telle que motifs polyoxyalkylènes ou polyglycérolés.

L'élastomère de silicone non émulsionnant sphérique est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP 295 886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (A2) peut avoir une structure chaîne ramifiée, chaîne linéaire, cyclique ou réseau mais la structure chaîne linéaire est préférée. Le composé (A2) peut avoir une viscosité allant de l'état liquide à l'état de gomme. De préférence, le composé (A2) a une viscosité d'au moins 100 centistokes à 25 °C.

Les organopolysiloxanes (A2) peuvent être choisi parmi les méthylvinylsiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2).

Avantageusement, la somme du nombre de groupements éthyléniques par molécule du composé (A2) et le nombre d'atomes d'hydrogène liés au silicum par molécule du composé (B2) est d'au moins 4.

Le composé (B2) peut être sous toute structure moléculaire, notamment de structure chaîne linéaire, chaîne ramifiée, structure cyclique.

Le composé (B2) peut avoir une viscosité à 25 °C allant de 1 à 50 000 centistokes, notamment pour être bien miscible avec le composé (A).

Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.

Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydro génosiloxane.

Le composé (C2) est le catalyseur de la réaction de réticulation, et est notamment l'acide chloroplatinique, les complexes acide chloroplatinique-oléfme, les complexes acide chloroplatinique-alkenylsiloxane, les complexes acide chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un groupe ester carboxylate, un groupe mercapto.

Les particules d'organopolysiloxane réticulés élastomères peuvent être véhiculées sous forme de gel constitué d'un organopolysiloxane élastomérique inclus dans au moins une huile hydrocarbonée et/ou une huile siliconée. Dans ces gels, les particules d'organopolysiloxanes sont souvent des particules non-sphériques.

Les particules d'organopolysiloxane réticulés élastomères peuvent également se présenter sous forme de poudre, notamment sous forme de poudre sphérique.

Des élastomères non-émulsionnants sphériques sont notamment décrits dans les demandes JP61194009, EP 242 219, EP 285 886, EP 765 656.

Comme élastomères non-émulsionnants sphériques, on peut utiliser ceux vendus sous les dénominations "DC 9040", "DC9041", "DC 9509", "DC9505", "DC 9506" par la société DOW CORNING.

L'élastomère de silicone non émulsionnant sphérique peut se présenter également sous forme de poudre d'organopolysiloxane réticulé élastomère enrobée de résine de silicone, notamment de résine silsesquioxane, comme décrit par exemple dans le brevet US 5 538 793. De tels élastomères sont vendus sous les dénominations "KSP-100", "KSP-101", "KSP-102", "KSP-103", KSP-104", "KSP-105" par la société SHIN ETSU.

D'autres organopolysiloxane réticulé élastomère sous forme de poudres sphériques peuvent être des poudres de silicone hybride fonctionnalisé par des groupes fluoroalkyle, notamment vendues sous la dénomination "KSP-200" par la société SHIN ETSU ; des poudres de silicones hybride fonctionnalisé par des groupes phényl, notamment vendues sous la dénomination "KSP-300" par la société SHIN ETSU.

L'élastomère de silicone sphérique non émulsionnant peut être présent dans la composition en une teneur allant de 0,1 % à 95 % en poids, par rapport au poids total de la composition, de préférence allant de 0,5 % à 75 % en poids, et préférentiellement allant de 1 % à 50 % en poids, plus préférentiellement allant de 1 % à 40 % en poids, et encore plus préférentiellement allant de 1 % à 30 % en poids.

### Agent hydratant

La composition selon l'invention peut comprendre un agent hydratant, en particulier un agent hydratant miscible à l'eau à 25 °C.

L'agent hydratant peut être notamment un alcool polyhydrique, en particulier choisi parmi les polyols ayant notamment de 2 à 20 atomes de carbone, de préférence ayant de 2 à 10 atomes de carbone, et préférentiellement ayant de 2 à 6 atomes de carbones. L'agent hydratant peut être par exemple choisi parmi la glycérine, le propylène glycol, le butylène glycol, le pentylène glycol, l'hexylène glycol, le dipropylène glycol, le diéthylène glycol, le sorbitol, l'hydroxypropyl sorbitol, le 1,2,6-hexanetriol ;les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol ; et leurs mélanges.

L'agent hydratant peut être présent dans la composition en une teneur allant de 1 % à 60 % en poids, par rapport au poids total de la composition, de préférence allant de 2 % à 40 % en poids, et préférentiellement allant de 3 % à 20 % en poids.

### Polymère filmogène

La composition selon l'invention peut comprendre un polymère filmogène.

Par polymère "filmogène", on entend un polymère apte à former à lui seul ou en présence d'un agent auxiliaire de filmification, un film continu et adhérent sur un support, notamment sur les matières kératiniques, et de préférence un film cohésif et mieux encore, un film dont la cohésion et les propriétés mécaniques sont telles que ledit film peut être isolé dudit support.

Dans un mode de réalisation, le polymère organique filmogène est au moins un polymère choisi parmi le groupe comprenant :
- les polymères filmogènes solubles dans un milieu liquide organique, en particulier les polymères liposolubles, lorsque le milieu liquide organique comprend au moins une huile ;
- les polymères filmogènes dispersibles dans un milieu solvant organique, en particulier les polymères sous la forme de dispersions non aqueuses de particules de polymères, de préférence des dispersions dans les huiles siliconées ou hydrocarbonées ; dans un mode de réalisation, les dispersions non aqueuses de polymère comprennent des particules de polymères stabilisées sur leur surface par au moins un agent stabilisant ;
- les polymères filmogènes sous forme de dispersions aqueuses de particules de polymère, souvent appelées « latex » ; dans ce cas, la composition comprend une phase aqueuse ;
- les polymères filmogènes hydrosolubles ; dans ce cas, la composition comprend une phase aqueuse.
   Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose, les polymères siliconés, en particulier les résines de silicones, les polymères acrylique greffés silicone.
   Des polymères filmogènes sont notamment décrits dans la demande de brevet internationale déposée sous le n° PCT/FR03/02849.
   Le polymère filmogène peut être un polymère séquencé éthylénique linéaire filmogène, avantageusement exempt de styrène. De préférence encore, le polymère séquencé comprend moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
   De préférence, le polymère séquencé a un indice de polydispersité I supérieur à 2.
   De préférence également, les première et seconde séquences sont incompatibles l'une avec l'autre.
   Par polymère "éthylénique", on entend un polymère obtenu par polymérisation de monomères comprenant une insaturation éthylénique.
   Par polymère "séquencé", on entend un polymère comprenant au moins 2 séquences distinctes, de préférence au moins 3 séquences distinctes.
   Un tel polymère séquencé est décrit dans la demande de brevet français déposée sous le n° 03 11337.
   Le polymère filmogène peut également être sous la forme d'une dispersion de particules, de préférence solides, d'un polymère éthylénique greffé dans une phase grasse liquide. Une telle dispersion est notamment décrite dans la demande de brevet internationale déposée sous le n° de dépôt PCT/FR03/03709.
   Par polymère greffé, on entend un polymère ayant un squelette comprenant au moins une chaîne latérale pendante ou située en bout de chaîne, et de préférence pendante.
   Avantageusement, le polymère éthylénique greffé comprend un squelette éthylénique insoluble dans ladite phase grasse liquide, et des chaînes latérales liées de manière covalente audit squelette et solubles dans ledit milieu de dispersion.
   Le polymère filmogène peut être présent dans la composition en une teneur allant de 0,1 % à 60 % en poids, par rapport au poids total de la composition, de préférence allant de 0,1 % à 50 % en poids, de préférence allant de 0,5 à 40 % en poids, préférentiellement allant de 0,5 % à 30 % en poids, plus préférentiellement allant de 0,5 % à 20 % en poids, et encore plus préférentiellement allant de 0,5 % à 10 % en poids.

### Filtres solaires

La composition selon l'invention peut comprendre un système photoprotecteur capable de filtrer le rayonnement UV.

Selon l'invention, le système photoprotecteur peut être constitué par un ou plusieurs filtres organiques et/ou un ou plusieurs (nano)pigments minéraux.

Les filtres organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4 367 390, EP 863 145, EP 517 104, EP 570 838, EP 796 851, EP 775 698, EP 878 469, EP 933 376, EP 507 691, EP 507 692, EP 790 243, EP 944 624 ; les dérivés de la benzophénone ; les dérivés de β, β -diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP 669 323 et US 2 463 264; les dérivés de l'acide p-aminobenzoïque (PABA) ; les dérivés de méthylène bis-(hydroxyphényl benzotriazole) tels que décrits dans les demandes US 5,237,071, US 5,166,355, GB 2 303 549, DE 197 26 184 et EP 893 119 ; les polymères filtres et silicones filtres tels que ceux décrits notamment dans la demande WO-93/04665 ; les dimères dérivés d'α-alkylstyrène tels que ceux décrits dans la demande de brevet DE 1 985 5649 ; les 4,4-diarylbutadiènes tels que décrits dans les demandes EP 0 967 200, DE 1 974 6654, DE 1 975 5649, EP-A-1 008 586, EP 1 133 980 et EP 1 133 981 et leurs mélanges.

Comme exemples de filtres organiques actifs dans l'UV-A et/ou l'UV-B, on peut citer désignés ci-dessus sous leur nom INCI :

### Dérivés de l'acide para-aminobenzoique

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques

- Homosalate vendu sous le nom « Eusolex HMS » par Rona/EM Industries,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

### Dérivés du dibenzoylméthane

- Butyl Methoxydibenzoylmethane vendu notamment sous le nom commercial « PARSOL 1789 » par HOFFMANN LAROCHE,
- Isopropyl Dibenzoylmethane,

### Dérivés cinnamiques

- Ethylhexyl Methoxycinnamate vendu notamment sous le nom commercial « PARSOL MCX » par HOFFMANN LAROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- Diisopropyl Methylcinnamate,
- Glyceryl Ethylhexanoate Dimethoxycinnamate

### Dérivés de β,β-diphénylacrylate

- Octocrylene vendu notamment sous le nom commercial « UVINUL N539 » par BASF,
- Etocrylene, vendu notamment sous le nom commercial « UVINUL N35 » par BASF,

### Dérivés de la benzophénone

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « Helisorb 11 » par NORQUAY
- Benzophenone-8 vendu sous le nom commercial « Spectra-Sorb UV-24 » par AMERICAN CYANAMID
- Benzophenone-9 vendu sous le nom commercial« UVINUL DS-49» par BASF,
- Benzophenone-12,
- le 2-(4-diéthylamino-2-hydroxybenzoyl)-benzoate de n-hexyle

### Dérivés du benzylidène camphre

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

### Dérivés de benzimidazole

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial « NEO HELIOPAN AP » par HAARMANN et REIMER,

### Dérivés de triazine

- Anisotriazine vendu sous le nom commercial «TINOSORB S » par CIBA SPECIALTY CHEMICALS
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V
- la 2,4,6- tris-(4'amino-benzalmalonate de diisobutyle)-s-triazine.

### Dérivés de benzotriazole

- Drometrizole Trisiloxane vendu sous le nom « Silatrizole » par RHODIA CHIMIE ,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques

- Menthyl anthranilate vendu sous le nom commercial commercial « NEO HELIOPAN MA » par HAARMANN et REIMER,

### Dérivés d'imidazolines

Ethylhexyl Dimethoxybenzylidene Dioxoimidazoline Propionate,

### Dérivés de benzalmalonate

- Polyorganosiloxane à fonctions benzalmalonate vendu sous la dénomination commerciale « PARSOL SLX » par HOFFMANN LAROCHE

### Dérivés de 4,4-diarylbutadiène

- -1,1-dicarboxy (2,2'-diméthyl-propyl)-4,4-diphénylbutadiène
- et leurs mélanges.

Les filtres organiques plus particulièrement préférés sont choisis parmi les composés suivants :
- Ethylhexyl Salicylate,
- Ethylhexyl Methoxycinnamate
- Octocrylene,
- Phenylbenzimidazole Sulfonic Acid,
- Benzophenone-3,
- Benzophenone-4,
- Benzophenone-5,
- le 2-(4-diéthylamino-2-hydroxybenzoyl)-benzoate de n-hexyle
- 4-Methylbenzylidene camphor,
- Terephthalylidene Dicamphor Sulfonic Acid,
- Disodium Phenyl Dibenzimidazole Tetra-sulfonate,
- la 2,4,6-tris-(4'-amino benzalmalonate de diisobutyle)-s-triazine
- Anisotriazine,
- Ethylhexyl triazone,
- Diethylhexyl Butamido Triazone,
- Methylène bis-Benzotriazolyl Tetramethylbutylphénol
- Drometrizole Trisiloxane
- 1,1-dicarboxy (2,2'-diméthyl-propyl)-4,4-diphénylbutadiène
- et leurs mélanges.

Les filtres inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP 518 772 et EP 518 773.

Le système photoprotecteur peut être présent dans la composition en une teneur allant de 0,1 % à 30 % en poids, par rapport au poids total de la composition, de préférence allant de 0,5 % à 20 % en poids, et préférentiellement allant de 0,5 % à 15 % en poids.

### Fibres

La composition peut comprendre des fibres.

Par "fibre", il faut comprendre un objet de longueur L et de diamètre D tel que L soit supérieur à D, et de préférence, très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 µm à 10 mm, de préférence de 0,1 mm à 5 mm et mieux de 0,3 mm à 3 mm. Leur section peut être comprise dans un cercle de diamètre allant de 2 nm à 500 µm, de préférence allant de 100 nm à 100 µm et mieux de 1 µm à 50 µm. Le poids ou titre des fibres est souvent donné en denier ou décitex et représente le poids en gramme pour 9 km de fil. De préférence, les fibres selon l'invention ont un titre choisi dans la gamme allant de 0,01 à 10 à deniers, de préférence de 0,1 à 2 deniers et mieux de 0,3 à 0,7 deniers.

De telles fibres sont notamment décrites dans la demande de brevet français déposée sous le n° 04 50074, les demandes FR 2 844 710, EP 1 201 221.

Les fibres peuvent être présentes dans la composition en une teneur allant de 0,1 % à 30 % en poids, par rapport au poids total de la composition, de préférence allant de 0,1 % à 20 % en poids, et préférentiellement allant de 0,1 % à 10 % en poids.

### Les huiles

La composition selon l'invention peut comprendre au moins une huile.

L'huile peut être choisie parmi les huiles hydrocarbonées, les huiles siliconées, les huiles fluorées.

L'huile peut être choisie parmi les huiles volatiles, les huiles non volatiles, et leurs mélanges.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Par huile siliconée, on entend une huile contenant au moins un atome de silicium, et notamment contenant des groupes Si-O.

Par huile fluorée, on entend une huile contenant au moins un atome de fluor.

La composition selon l'invention peut comprendre au moins une huile volatile.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10-3 à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

En outre, l'huile volatile a généralement un point d'ébullition, mesuré à pression atmosphérique, allant de 150 °C à 260 °C, et de préférence allant de 170 °C à 250 °C.

La composition selon l'invention peut comprendre une huile volatile hydrocarbonée notamment choisie parmi les huiles hydrocarbonées ayant un point éclair allant de 40 °C à 102 °C, de préférence allant de 40 °C à 55 °C, et préférentiellement allant de 40 °C à 50 °C.

Comme huile volatile hydrocarbonée, on peut citer les huiles volatiles hydrocarbonées ayant de 8 à 16 atomes de carbone et leurs mélanges, et notamment les alcanes ramifiés en C₈-C₁₆ comme les iso-alcanes (appelées aussi isoparaffines) en C₈-C₁₆, l'isododécane, fisodécane, l'isohexadécane et par exemple les huiles vendues sous les noms commerciaux d'Isopars ou de Permetyls, les esters ramifiés en C₈-C₁₆ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

Comme huile volatile siliconée, on peut citer les silicones linéaires ou cycliques ayant de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

L'huile volatile peut être présente dans la composition selon l'invention en une teneur allant de 0,1 % à 90 % en poids, par rapport au poids total de la composition, de préférence allant de 1 % à 70 % en poids, et préférentiellement allant de 5 % à 50 % en poids.

La composition selon l'invention peut comprendre au moins une huile non volatile.

Comme huile non volatile hydrocarbonée, on peut utiliser l'huile de paraffine (ou vaseline), le squalane, le polyisobutylène hydrogéné (huile de Parléam), le perhydrosqualène, l'huile de vison, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, notamment en C₁₂-C₃₆, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le trüsostéarate de glycérine ou de diglycérine ; l'acide béhénique, l'acide oléique, l'acide linoléique, l'acide linolénique ou l'acide isostéarique ; les alcools gras supérieurs, notamment en C₁₆- C₂₂, tels que le cétanol, l'alcool oléique, l'alcool linoléique ou linolénique, l'alcool isostéarique ou l'octyl dodécanol ; et leurs mélanges.

L'huile non volatile peut être présente en une teneur allant de 0,1 % à 70 % en poids, par rapport au poids total de la phase grasse liquide non volatile, de préférence allant de 0,5 % à 60 % en poids, et préférentiellement allant de 1 % à 50 % en poids.

### Agents structurants

La composition selon l'invention peut comprendre un agent structurant.

On entend par agent structurant un composé apte à augmenter la viscosité de la composition. L'agent structurant permet notamment d'obtenir une composition pouvant présenter une texture allant des textures fluides à solides.

L'agent structurant peut être présent dans la composition en une teneur allant de 0,1 % à 40 % en poids, par rapport au poids total de la composition, de préférence allant de 0,1 % à 30 % en poids, et préférentiellement allant de 0,5 % à 25 % en poids.

L'agent structurant peut être notamment choisi parmi les épaississants (épaississants de milieux huileux ; épaississants de milieu aqueux), les organogélateurs, les cires, les composés pâteux, les gommes.

L'agent épaississant de milieu aqueux peut être choisi parmi :
- les argiles hydrophiles,
- la silice pyrogénée hydrophile.
- les épaississants cellulosiques hydrosolubles
- les gommes de guar, de xanthane, de caroube, de scléroglucane, de gellane, de rhamsan, de karaya, de carraghénane
- les alginates, les maltodextrines, l'amidon et ses dérivés, l'acide hyaluronique et ses sels,
- les polymères poly(métha)crylates de glycéryle vendus sous les dénominations de "Hispagel" ou "Lubragel" par les Sociétés HISPANO QUIMICA ou GUARDIAN,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société HOECHST, de "Sepigel 305" par la Société SEPPIN par la Société ALLIED COLLOÏD, ou encore
- les homopolymères réticulés de chlorure de méthacryloyloxyéthyltriméthylammonium vendus sous la dénomination de "Salcare SC95" par la Société ALLIED COLLOÏD.
- les polymères associatifs et notamment les polyuréthanes associatifs.

De tels agents épaississants sont notamment décrits dans les demandes EP 1 400 234.

L'agent épaississant de milieu huileux peut être choisi parmi
- les argiles organophiles,
- les silices pyrogénées hydrophobes
- les gommes de guar alkylées (avec groupe alkyle en C₁-C₆), telles que celles décrites dans EP 708 114 ;
- les polymères gélifiant d'huile comme les polymères tribloc ou en étoile résultant de la polymérisation ou copolymérisation d'au moins monomère à groupe éthylénique, comme les polymères vendus sous la dénomination Kraton ;
- les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-02/056847, WO-02/47619 ; en particulier les résines de polyamides (notamment comprenant des groupes alkyles ayant de 12 à 22 atomes de carbone) telles que celles décrites dans US 5 783 657.
- les résines de polyamides siliconées telles que décrites dans la demande EP 1 266 647, dans la demande de brevet français déposée sous le n° 02 16039.

De tels agents épaississants sont notamment décrits dans les demandes EP 1 400 234.

Les organogélateurs peuvent être choisis parmi ceux décrits dans la demande WO-03/105788.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

La cire peut également présenter une dureté allant de 0,05 MPa à 15 MPa, et de préférence allant de 6 MPa à 15 MPa. La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Les cires peuvent être hydrocarbonées, fluorées et/ou siliconées et être d'origine végétale, minérale, animale et/ou synthétique. En particulier, les cires présentent une température de fusion supérieure à 30 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

A titre indicatif, la composition peut contenir de 0,1 à 50 % en poids de cires, par rapport au poids total de la composition et mieux de 1 à 30 % en poids.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

### Phase aqueuse

La composition peut ainsi comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en C₂ et des aldéhydes en C₂-C₄ hydrophiles.

L'eau ou le mélange d'eau et de solvants organiques hydrophiles peut être présent dans la composition selon l'invention en une teneur allant de 0,1 % à 95 % en poids, par rapport au poids total de la composition, et de préférence allant de 10 % à 80 % en poids.

### Tensioactif non réticulé

La composition selon l'invention peut comprendre au moins un tensioactif non réticulé à l'exclusion des tensioactifs siliconés non réticulés polyglycérolés.

Le tensioactif non réticulé peut être choisi parmi les tensioactifs non réticulés non ioniques, anioniques, cationiques, amphotères.

Le tensioactif non réticulé non ionique peut être choisis parmi :
- un alkyl C₈-C₂₂ diméthicone copolyol, c'est-à-dire un poly méthyl alkyl(C₈-C₂₂) diméthyl méthyl siloxane oxypropyléné et/ou oxyéthyléné.

L'alkyl C₈-C₂₂ diméthicone copolyol est avantageusement un composé de formule (I) suivante : 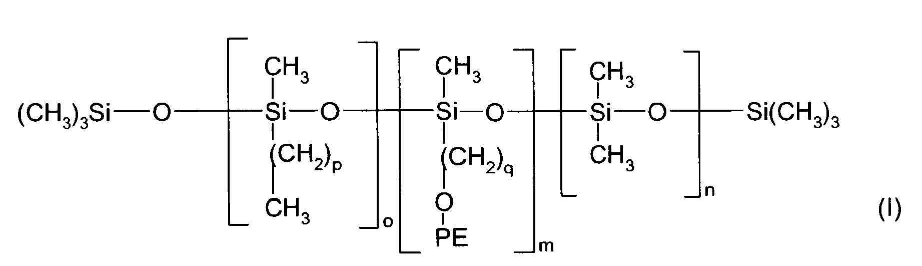 dans laquelle :
- PE représente (-C₂H₄O)x-(C₃H₆O)y-R, R étant choisi parmi un atome d'hydrogène et un radical alkyle de 1 à 4 atomes de carbone, x allant de 0 à 100 et y allant de 0 à 80, x et y n'étant pas simultanément 0
- m allant de 1 à 40
- n allant de 10 à 200
- o allant de 1 à 100
- p allant de 7 et 21
- q allant de 0 à 4
et de préférence :
- R=H
- m =1 à 10
- n = 10 à 100
- o = 1 à 30
- p = 15
- q = 3

Comme alkyl C₈-C₂₂ diméthicone copolyol, on peut citer le cétyl diméthicone copolyol comme le produit commercialisé sous la dénomination Abil EM-90 par la société GOLDSCHMIDT.
- un diméthicone copolyol, c'est-à-dire un polydiméthyl méthyl siloxane oxypropyléné et/ou oxyéthyléné. Il ne contient pas de groupement alkyle à longue chaîne de plus de 8 atomes de carbone, notamment en C₈-C₂₂,

On peut utiliser comme diméthicone copolyol ceux répondant à la formule (II) suivante : 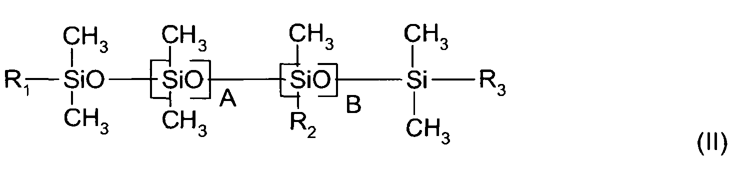 dans laquelle :
- R₁, R₂, R₃, indépendamment les uns des autres, représentent un radical alkyle en C₁-C₆ ou un radical -(CH₂)x - (OCH₂CH₂)y - (OCH₂CH₂CH₂)z - OR₄, au moins un radical R₁, R₂ ou R₃ n'étant pas un radical alkyle ; R₄ étant un hydrogène, un radical alkyle en C₁-C₃ ou un radical acyle en C₂-C₄ ;
- A est un nombre entier allant de 0 à 200 ;
- B est un nombre entier allant de 0 à 50 ; à la condition que A et B ne soient pas égaux à zéro en même temps ;
- x est un nombre entier allant de 1 à 6 ;
- y est un nombre entier allant de 1 à 30 ;
- z est un nombre entier allant de 0 à 5.
   Selon un mode de réalisation préféré de l'invention, dans le composé de formule (II), R₁ = R₃ = radical méthyle, x est un nombre entier allant de 2 à 6 et y est un nombre entier allant de 4 à 30. R4 est en particulier un hydrogène.
   On peut citer, à titre d'exemple composés de formule (II), les composés de formule (III) : 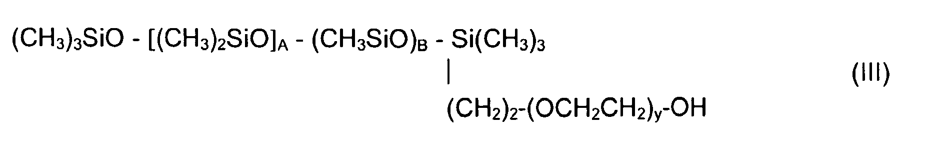
dans laquelle A est un nombre entier allant de 20 à 105, B est un nombre entier allant de 2 à 10 et y est un nombre entier allant de 10 à 20.

On peut également citer à titre d'exemple de composés siliconés de formule (II), les composés de formule (IV) :

HO - (CH₂CH₂O)y-(CH₂)₃ - [(CH₃)₂SiO]A' - [(CH₃)₂Si] - (CH₂)₃ -(OCH₂CH₂)y - OH (IV)

dans laquelle A' et y sont des nombres entiers allant de 10 à 20.

On peut utiliser comme diméthicone copolyol ceux vendus sous les dénominations DC 5329, DC 7439-146, DC 2-5695, Q4-3667 par la société DOW CORNING ; KF-6013, KF-6015, KF-6016, KF-6017 par la société SHIN ETSU.

Les composés DC 5329, DC 7439-146, DC 2-5695 sont des composés de formule (III) où respectivement A est 22, B est 2 et y est 12 ; A est 103, B est 10 et y est 12 ; A est 27, B est 3 et y est 12.

Comme tensioactif non réticulé non ionique, on peut également citer les esters d'acides gras de polyols comme les mono-, di-, tri- ou sesqui-oléates ou stéarates de sorbitol ou de glycérol, les laurates de glycérol ou de polyéthylène glycol ; les esters d'acides gras de polyéthylène glycol (monostéarate ou monolaurate de polyéthylène glycol) ; les esters d'acides gras (stéarate, oléate) de sorbitol polyoxyéthylénés ; les alkyl (lauryl, cétyl, stéaryl, octyl) éthers polyoxyéthylénés.

Comme tensioactifs anioniques, on peut citer les carboxylates (2-(2-Hydroxyalkyloxy) acétate de sodium), les dérivés des aminoacides (N-acylglutamates, N-acylglycinates, acylsarcosinates), les alkyl sulfates, les alkyl éther sulfates et leurs dérivés oxyéthylénés, les sulfonates, les iséthionates et N-acyliséthionates, les taurates et N-acyl N-méthyltaurates, les sulfosuccinates, les alkylsulfoacétates, les phosphates et alkylphosphates, les polypeptides, les dérivés anioniques d'alkyl polyglycoside (acyl-D-galactoside uronate), les savons d'acides gras, et leurs mélanges.

Comme tensioactifs amphotères et zwitterioniques , on peut utiliser les bétaïnes, les N-alkylamidobéta'ines et leurs dérivés, les dérivés de la glycine, les sultaïnes, les alkyl polyaminocarboxylates, les alkylamphoacétates, et leurs mélanges.

De tels tensioactifs sont notamment décrits dans la demande WO-02/056854.

Le tensioactif non réticulé peut être présent dans la composition selon l'invention en une teneur allant de 0,1 % à 10 % en poids, par rapport au poids total de la composition, et de préférence allant de 0,5 % à 8 % en poids, et préférentiellement allant de 1 % à 7 % en poids.

### Charges

La composition selon l'invention peut comprendre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide z(Nylon^{®}) (Orgasol^{®} de chez ATOCHEM), de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (NOBEL INDUSTRIE), de copolymères d'acide acrylique (Polytrap^{®} de la société DOW CORNING) et les microbilles de résine de silicone (Tospearls^{®} de TOSHIBA, par exemple), les particules de polyorganosiloxanes élastomères, le carbonate de calcium précipité, le carbonate et l'hydrocarbonate de magnésium, l'hydroxyapatite, les microsphères de silice creuses (Silica Beads^{®} de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

### Pigment enrobés

La composition peut comprendre des pigments traités avec un agent hydrophobe. L'agent de traitement hydrophobe peut être choisi parmi les silicones comme les méthicones, les diméthicones, les perfluoroalkylsilanes, les perfluoroalkyl silazanes, le triéthoxy caprylylsilane, le triéthoxysilyléthyl polydiméthylsiloxyéthyl hexyl diméthicone ; les acides gras comme l'acide stéarique ; les savons métalliques comme le dimyristate d'aluminium, le sel d'aluminium du glutamate de suif hydrogéné ; les perfluoroalkyl phosphates, les polyoxydes d'hexafluoropropylène, les polyorganosiloxanes comprenant des groupes perfluoroalkylles perfluoropolyéthers, les polymères acryliques greffés silicone (notamment décrits dans la demande JP05339125 ; les acides aminés ; les acides aminés N-acylés ou leurs sels ; la lécithine, le trisostéaryle titanate d'isopropyle, le sébaçate d'isostéaryle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine.

Le terme alkyl mentionné dans les composés cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP 1 086 683.

Les colorants hydrosolubles sont par exemple le jus de betterave, le bleu de méthylène.

Les matières colorantes, en particulier les pigments traités avec un agent hydrophobe, peuvent être présents dans la composition en une teneur allant de 0,1 % à 50 % en poids, par rapport au poids total de la composition, de préférence allant de 0,5 % à 30 % en poids, et préférentiellement allant de 1 % à 20 % en poids.

### Matière colorante

La composition peut comporter au moins une matière colorante additionnelle, outre les particules fluorescentes. L'expression « matière colorante » désigne toute substance organique ou inorganique capable de procurer un effet coloré.

La matière colorante additionnelle peut être présente à raison de 0,01 à 20 % en poids, notamment de 0,05 à 10 % en poids, par exemple de 0,1 à 7 % en poids, par exemple de 0,1 à 5 % en poids, par rapport au poids total de la composition.

A titre d'exemple de matières colorantes, figurent les colorants liposolubles, les colorants hydrosolubles, les pigments et les nacres habituellement utilisés dans les compositions cosmétiques ou dermatologiques, et leurs mélanges.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les pigments peuvent être blancs ou colorés, minéraux et/ou organiques, enrobés comme décrit précédemment ou non.

Les pigments peuvent être choisis parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome, le bleu ferrique, et les poudres métalliques comme la poudre d'aluminium et la poudre de cuivre.

Parmi les pigments organiques, on peut citer le noir de carbone, les pigments répertoriés selon la classification D&C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les pigments peuvent avoir subi un traitement de surface.

Des pigments à effets peuvent encore être utilisés, notamment des pigments interférentiels.

Le choix de la matière colorante additionnelle peut se faire en fonction de la ou des longueurs d'onde de photoluminescence, lorsque par exemple la photoluminescence est mise à profit pour renforcer la couleur de la matière additionnelle.

La composition selon l'invention peut également contenir des ingrédients couramment utilisés en cosmétique, tels que les vitamines, les épaississants, les oligoéléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, ou leurs mélanges.

Bien entendu, l'homme du métier veillera à choisir ce ou ces éventuels composés complémentaires, et/ou leur quantité, de manière telle que les propriétés recherchées de la composition selon l'invention ne soient pas, ou substantiellement pas, altérées par l'adjonction envisagée.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

### Dispositifs de conditionnement et/ou d'application

La composition peut être conditionnée sous de multiples formes, avec un applicateur ou non, selon la forme galénique.

La composition peut être conditionnée dans un dispositif de conditionnement, tel qu'un boîtier, récipient ou étui, étanche au moins avant la première utilisation. Ce dispositif de conditionnement peut être réalisé au moins partiellement avec au moins une matière thermoplastique ou en variante sans aucune matière thermoplastique. Le dispositif de conditionnement peut comporter une polyoléfine. Le dispositif de conditionnement peut également comporter au moins un élément métallique, par exemple une coupelle, une âme métallique torsadée, une charnière, une bague, un capot.

Lorsque la composition est destinée à être appliquée au moyen d'un applicateur, l'applicateur comporte par exemple une mousse, un embout floqué ou non, un feutre, une brosse, un peigne, un pinceau, un tissé, un non-tissé.

La composition peut encore imprégner un substrat, par exemple un papier, un tissé ou un non-tissé.

Lorsque présent, l'applicateur peut se loger de manière amovible sur le dispositif de conditionnement contenant la composition. En variante, l'applicateur peut être fixé à demeure sur le dispositif de conditionnement contenant la composition. Le dispositif de conditionnement peut comporter un piston ou tout autre moyen pour permettre l'alimentation de l'applicateur avec la composition.

Le dispositif de conditionnement peut comporter un organe de distribution tel qu'une pompe ou une valve, notamment lorsque la composition est liquide.

Lorsque présent, l'applicateur peut comporter une tige reliée à un organe de fermeture du dispositif de conditionnement, cet organe de fermeture pouvant également constituer un organe de préhension, le cas échéant.

Le dispositif de conditionnement contenant la composition peut être pourvu d'un fermoir ou autre moyen de fermeture, par exemple magnétique ou à encliquetage.

Le dispositif de conditionnement peut encore être pourvu d'un moyen de fermeture se fixant par vissage, friction ou encliquetage.

Le dispositif de conditionnement peut comporter des moyens d'étanchéité tels que par exemple une lèvre annulaire d'étanchéité ou un joint en élastomère, surinjecté ou rapporté.

Le dispositif de conditionnement contenant la composition peut comporter une étiquette ou une impression indiquant par exemple une marque ou un logo, cette impression ayant par exemple été réalisée par transfert à chaud ou à froid ou par sérigraphie ou par d'autres techniques d'impression.

Le dispositif de conditionnement contenant la composition peut comporter un emballage cartonné ou un blister, par exemple au moins partiellement réalisé en matière plastique transparente.

### Procédé de maquillage

L'invention a encore pour objet un procédé de maquillage et/ou de soin non thérapeutique de la peau, des lèvres et/ou des fibres kératiniques comprenant l'étape d'application sur la peau, les lèvres et/ou les fibres kératiniques d'une composition telle que définie plus haut. Préalablement, une mesure de propriétés optiques de la peau peut être effectuée, afin de formuler la composition en conséquence.

### Milieu physiologiquement acceptable

La composition selon l'invention comprend au moins un milieu physiologiquement acceptable.

Par « milieu physiologiquement acceptable », on désigne un milieu non toxique et susceptible d'être appliqué sur la peau, les lèvres ou matières kératiniques d'êtres humains. Le milieu physiologiquement acceptable est généralement adapté à la nature du support sur lequel doit être appliquée la composition ainsi qu'à l'aspect sous lequel la composition est destinée à être conditionnée.

La composition de l'invention peut dans certains exemples de mise en oeuvre comprendre une phase grasse, notamment à raison de 5 à 80 % en poids, et en particulier de 5 à 50 %, en poids par rapport au poids total de la composition.

Les huiles qui peuvent être utilisées dans la composition peuvent être choisies parmi celles classiquement utilisées dans le domaine considéré.

La composition peut comprendre une huile, notamment choisie parmi,
- les huiles hydrocarbonées d'origine animale, telles que le perhydrosqualène,
- les huiles hydrocarbonées d'origine végétale, telles que les triglycérides liquides d'acides gras comportant de 4 à 10 atomes de carbone et la fraction liquide du beurre de karité,
- les esters et les éthers de synthèse, notamment d'acides gras, comme les huiles de formules R₁COOR₂ et R₁OR₂ dans laquelle R₁ représente le reste d'un acide gras ou d'un alcool gras comportant de 8 à 29 atomes de carbone, et R₂ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle,
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam,
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique,
- les huiles fluorées partiellement hydrocarbonées et/ou siliconées comme celles décrites dans le document JP2295912,
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; et
- leurs mélanges.

Dans le cas où la composition se présente sous la forme d'une émulsion à phase grasse, elle peut en outre comprendre un émulsionnant, et éventuellement un coémulsionnant.

Comme émulsionnants et coémulsionnants utilisables dans l'invention, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H tels que le poly(méthylcétyl)(diméthyl)méthylsiloxane oxyéthyléné disponible sous la dénomination commerciale Abil WE09 auprès de la société DEGUSSA GOLDSCHMIDT ou le mélange de stéarate d'éthylène glycol acétyle et de tristéarate de glycéryle commercialisé par la société GUARDIAN sous la dénomination commerciale Unitwix. On peut également citer les émulsionnants tels que les élastomères de silicone décrits précédemment.

L'émulsionnant et éventuellement le coémulsionnant sont généralement présents, dans la composition, en une proportion allant de 0,3 à 30 % en poids, et en particulier de 0,5 à 20 % en poids par rapport au poids total de la composition.

La composition selon l'invention peut également comprendre au moins une cire, au moins une gomme et/ou au moins un corps gras pâteux, d'origine végétale, animale, minérale ou de synthèse, siliconé ou non.

Les cires peuvent être hydrocarbonées, siliconées et/ou fluorées, composées éventuellement des fonctions ester ou hydroxyle. Elles peuvent être notamment d'origine naturelle.

La cire peut représenter de 0,01 à 10 % en poids, notamment de 0,1 à 5 % en poids, par rapport au poids total de la composition. Selon un mode de réalisation, la composition peut être exempte de cire.

La composition peut également comprendre au moins une charge minérale ou organique, enrobée ou non, notamment à titre d'agent matifiant, par exemple l'oxyde de zinc et de zircone, la silice, l'alumine, le nitrure de bore, le talc, la séricite, le mica, les argiles, l'amidon et ses dérivés, notamment l'amidon réticulé par l'anhydride octénylsuccinique commercialisé par la société NATIONAL STARCH sous la dénomination DRY FLO PLUS (28-1160), les dispersions aqueuses de styrène acrylique, les particules de résine de mélamine-formaldéhyde ou d'urée-formaldéhyde, les dispersions aqueuses de polytétrafluoroéthylène, les microdispersions de cires, les copolymères vinylpyrrolidone/1-triacontène, les polymères hydrodispersibles contenant des unités à LCST, les cires et résines de silicone, notamment les microbilles de résine de silicone telles que celles commercialisées sous la dénomination TOSPEARL par la société TOSHIBA SILICONE, les poudres expansées telles que les microsphères creuses et notamment les microsphères commercialisées sous la dénomination EXPANCEL par la société KEMANORD PLAST ou sous la dénomination MICROPEARL F 80 ED par la société MATSUMOTO, les microsphères de terpolymère expansé de chlorure de vinylidène, d'acrylonitrile et de méthacrylate, les particules de polyamide, par exemple de Nylon^{®} ou celles vendues sous la dénomination ORGASOL par la société ATOCHEM, les microbilles de cellulose, les fibres, les poudres de polyéthylène, les microsphères à base de copolymères acryliques, telles que celles en copolymère diméthacrylate d'éthylène glycol/ méthacrylate de lauryle vendues par la société DOW CORNING sous la dénomination de POLYTRAP et leurs mélanges.

La charge, notamment lorsqu'utilisée comme agent matifiant, peut être présente en une teneur allant de 0,1 à 80 % en poids par rapport au poids total de la composition, par exemple une teneur comprise entre 0,1 % et 10 % en poids par rapport au poids total de la composition.

Il est bien entendu que le choix de cet agent matifiant, de même que celui de la quantité utilisée, seront effectués par l'homme du métier de manière à ne pas nuire aux propriétés recherchées.

La composition peut contenir également au moins un adjuvant habituel dans le domaine cosmétique, tel que les charges, par exemple choisies dans la liste précitée, les gélifiants hydrophiles ou lipophiles, les actifs, hydrosolubles ou liposolubles, les conservateurs, les hydratants, tels que les polyols et notamment la glycérine, les séquestrants, les antioxydants, les solvants, les parfums, les filtres solaires physiques et chimiques, notamment aux UVA et/ou aux UVB, les absorbeurs d'odeur, les ajusteurs de pH (acides ou bases) et leurs mélanges.

Les quantités de ces différents adjuvants sont celles classiquement utilisées dans le domaine considéré, par exemple de 0,01 à 20 % du poids total de la composition.

En tout état de cause, ces adjuvants, ainsi que leurs proportions, seront choisis de manière à ne pas nuire aux propriétés recherchées selon l'invention.

A titre d'actifs, on peut citer en particulier :
- les actifs connus pour leur activité sur le vieillissement de la peau comme les agents keratolytiques ou prodesquamants, par exemple les α-hydroxy-acides, les β-hydroxyacides, les α-céto-acides, les β-céto-acides, les rétinoïdes et leurs esters, le rétinal, l'acide rétinoïque et ses dérivés,
- les vitamines, telles que par exemple les vitamines A, B3, PP, B5, E, K1 et/ou C, et les dérivés de ces vitamines et notamment leurs esters,
- les agents anti-radicaux libres,
- les filtres solaires,
- les agents hydratants comme les polyols,
- les céramides,
- la DHEA et ses dérivés,
- le coenzyme Q10,
- les agents blanchissants et dépigmentants par action biologique complémentaires comme l'acide kojique, les extraits de scutellaire, de mûrier, de réglisse et/ou de camomille, les dérivés de para-aminophénols, l'arbutine et leurs dérivés, et leurs mélanges,
- les actifs utiles pour les peaux grasses ou mixtes, tels que les sels de zinc et en particulier l'oxyde de zinc et le gluconate de zinc,
- les antibactériens comme l'acide salicylique et ses dérivés tels que l'acide n-octanoyl-5-salicylique, le triclosan, le lipacide, la capryloylglycine, l'extrait de clou de girofle, l'octopirox, l'hexamidine, l'acide azélaïque et ses dérivés,
- les actifs anti-acné, ou encore,
- les extraits de plantes veinotoniques tels que les extraits de petit houx et/ou de marron d'Inde ; les bases xanthiques telles que la caféine.

La composition selon l'invention peut se présenter sous toutes les formes galéniques normalement utilisées dans le domaine cosmétique, normalement utilisées pour une application topique : émulsions directes, inverses ou multiples, gels, crèmes, solutions, suspensions, lotions, poudres libres, compactes et sticks.

Elle peut plus précisément se présenter sous forme de solution huileuse éventuellement gélifiée, d'émulsion de consistance liquide ou semi-liquide du type lait, obtenue par dispersion d'une phase grasse dans une phase aqueuse (H/E) ou inversement (E/H), d'une émulsion triple (E/H/E ou H/E/H), ou de suspension ou émulsion de consistance molle, semi-solide ou solide de type crème ou gel, ou encore de microémulsions, de microcapsules, de microparticules ou d'une dispersion vésiculaire de type ionique (liposomes ou oléosomes) et/ou non ionique (niosomes) et/ou d'une dispersion de nanocapsules ou nanosphères.

La composition cosmétique selon l'invention peut notamment se présenter sous forme de composition de soin, de maquillage et/ou de protection solaire.

Plus particulièrement, la composition selon l'invention se présente sous la forme d'un produit de maquillage du visage, notamment de la peau et/ou des lèvres, par exemple sous forme de fond de teint.

La composition selon l'invention peut encore se présenter sous la forme d'une composition de soin et/ou de maquillage de la peau, des lèvres et/ou des fibres kératiniques.

La composition peut par exemple se présenter sous forme de gel anti-cernes, de crème de soin ou de lotion photoprotectrice, notamment des UV.

### Exemples proposés

Les proportions sont massiques sauf indications contraires.

### Exemple 1 : synthèse de la matrice contenant une molécule fluorescente

On a mélangé d'abord 60g d'isopropanol, 57.5g de tetraméthoxysilane (TMOS) et 0.6g de Tinopal CBX (azurant optique vendu par la société CIBA SPECIALTY CHEMICALS), puis on rajoute 102g d'acide phosphorique à 0.01N (0.005 Mole/L) et on laisse 24h sous agitation à 25°C afin d'obtenir un sol.

Le sol est ensuite appliqué sur une plaque en acier à l'aide d'un spin-coater (vitesse de rotation 600rpm) pour former un film, laissé 30 secondes à température ambiante puis séché à 200°C pendant 60 secondes. Le film obtenu est ensuite cassé pour obtenir des plaquettes ayant une épaisseur de 1 µm et une taille moyenne de 5-50 µm Ces particules contiennent 2.5% en poids de Tinopal CBX et sont de couleur blanche.

Ces particules présentent une forte fluorescence, avec un maximum d'émission à 440nm.

### Exemple 2 et Exemples comparatifs 3 et 4

Les formulations suivantes ont été réalisées.

| | Exemple 2 | Exemple 3 (comparatif) | Exemple 4 (comparatif) |
|---|---|---|---|
| Eau ultrapure | 99 | 99 | 99 |
| Particule selon l'exemple 1 | 1 | | |
| Dermaglo DG-00¹ | | 1 | |
| Colment Crazy Blue Azur² | | | 1 |
| Quantité de Tinopal CBX présente en solution aqueuse (mg/L) après une heure à une température de 25° C. | 0.5 | 147 | 250 |

| | | | |
|---|---|---|---|
| ¹ particules de polyester contenant 3% de Tinopal CBX commercialisé par la société DAYGLO ² particules de PMMA contenant 2.5% de Tinopal CBX commercialisé par la société LCW | | | |

Afin de mesurer la quantité de Tinopal CBX présente dans la solution aqueuse en dehors des particules, on a utilisé la procédure suivante : les solutions ont été centrifugées pendant 1h à 4000rpm, puis le surnageant a été recueilli et centrifugé à nouveau pendant 1 h à 4000rpm. Le surnageant final est dilué d'un facteur 10 par de l'eau ultrapure et l'intensité de fluorescence a ensuite été mesurée à l'aide du spectrofluorimètre JASCO FP6000. La concentration est alors déterminée par comparaison avec une courbe d'étalonnage du Tinopal CBX en solution aqueuse.

### Exemple 5 : (Fond de teint liquide)

**Phase I**

| | |
|---|---|
| Cetyl dimethicone copolyol/ | |
| polyglyceryl-4 Isostearate/hexyl laurate | 8g |
| Dimethicone | 4.8g |
| Cyclomethicone | 5.2g |
| Isododécane | 2.8g |
| Isostearyl Neopentanoate | 0.8g |
| Bentone gel | 8g |
| Dioxyde de titane | 5g |
| Oxyde de fer | 1g |

**Phase II**

| | |
|---|---|
| Eau | 43.2g |
| Butylène glycol | 5.6g |
| Sulfate de Magnésium | 0.8g |
| Conservateurs | 0.8g |

**Phase III**

| | |
|---|---|
| Talc | 4g |
| Particules selon l'exemple 1 | 10g |

Les phases I et II sont mélangées séparément, la phase II est ensuite rajoutée à la phase I à l'aide d'une turbine adéquate pour réaliser l'émulsification. La phase III est ensuite rajoutée à l'émulsion.

### Exemple 6 : (Vernis à ongles)

| | |
|---|---|
| Nitrocellulose | 20g |
| N ethyl sulfonamide o,p toluene | 6g |
| Tributyle acetyl citrate | 6g |
| Hectorite | 1g |
| Particules selon l'exemple 1 | 10g |
| Isopropanol | 8g |
| Acetate d'ethyle/ Acetate de butyle | quantité suffisante pour 100g |

## Revendications

1. Composition cosmétique contenant dans un milieu physiologiquement acceptable, des particules fluorescentes comprenant des molécules d'au moins un composé organique fluorescent encapsulées à l'intérieur d'une matrice formée au moins en partie d'au moins un oxyde métallique et de phosphore.

2. Composition cosmétique selon la revendication 1 **caractérisée en ce que** le ou les composés organiques fluorescents sont choisis parmi les fluorescéines, les pyrazines, les coumarines, les naphtalimides, les triazines, les dioxazines, les sulforhodamines, les azoiques, les azomethiniques, les dérivés du stilbene, les dérivés oxazole, benzoxazole, l'imidazole, ou leurs mélanges.

3. Composition cosmétique selon l'une quelconque des revendications 1 ou 2 **caractérisée en ce que** le ou les oxydes métalliques sont choisis parmi les oxydes de silicium, de titane, d'aluminium, de zirconium, ou leurs mélanges.

4. Composition cosmétique selon la revendication précédente **caractérisée en ce que** le ou les oxydes métalliques sont choisis parmi les oxydes de silicium.

5. Composition cosmétique selon l'une quelconque des revendications précédentes **caractérisée en ce que** la phase liquide contient au moins un solvant volatil, notamment de l'eau, un alcool, ou une huile.

6. Composition cosmétique selon l'une quelconque des revendications précédentes **caractérisée en ce que** lesdites particules fluorescentes sont présentes en une teneur en poids allant de 0,5% à 95%, et de préférence, de 1% à 70%, et de préférence encore, de 5 à 30% par rapport au poids total de la composition.

7. Composition cosmétique selon l'une quelconque des revendications précédentes **caractérisée en ce qu'**elle comprend au moins une matière colorante additionnelle, de préférence diffusante, choisie notamment parmi le dioxyde de titane ou l'oxyde de zinc.

8. Composition cosmétique selon l'une quelconque des revendications précédentes **caractérisée en ce qu'**elle comprend en outre au moins une matière pulvérulente additionnelle choisie notamment parmi le talc, le mica, la silice, le polyamide, le polymethylméthacrylate.

9. Composition cosmétique selon l'une quelconque des revendications qui précédent **caractérisée en ce que** lesdites particules fluorescentes ont une taille moyenne en volume allant de 0,1 µm à 50 µm, et de préférence, de 0,5 µm à 20µm.

10. Procédé de maquillage et/ou de soin des matières kératiniques consistant à appliquer sur ces dernières une composition cosmétique selon l'une quelconque des revendications qui précèdent.

11. Procédé selon la revendication précédente **caractérisé en ce que** les matières kératiniques sont choisies parmi les cils, les ongles, la peau, les cheveux ou les lèvres.

## Claims

1. A cosmetic composition containing, in a physiologically acceptable medium, fluorescent particles comprising molecules of at least one fluorescent organic compound trapped inside a matrix at least partially formed by at least one metal oxide and phosphorous.

2. A cosmetic composition according to claim 1, **characterized in that** the fluorescent organic compound or compounds are selected from fluoresceins, pyrazines, coumarins, naphthalimides, triazines, dioxazines, sulforhodamines, azo compounds, azomethinic compounds, stilbene derivatives, oxazole derivatives, benzoxazole, imidazole or mixtures thereof.

3. A cosmetic composition according to claim 1 or claim 2, **characterized in that** the metal oxide or oxides are selected from oxides of silicon, titanium, aluminum, zirconium or mixtures thereof.

4. A cosmetic composition according to the preceding claim, **characterized in that** the metal oxide or oxides are selected from oxides of silicon.

5. A cosmetic composition according to any preceding claim, **characterized in that** the liquid phase contains at least one volatile solvent, in particular water, an alcohol or an oil.

6. cosmetic composition according to any preceding claim, **characterized in that** said fluorescent particles are present in an amount of 0.5% to 95% by weight, preferably 1% to 70% by weight and more preferably 5% to 30% by weight with respect to the total composition weight.

7. A cosmetic composition according to any preceding claim, **characterized in that** it comprises at least one additional coloring substance, preferably diffusing, selected from titanium dioxide and zinc oxide.

8. A cosmetic composition according to any preceding claim, **characterized in that** it further comprises at least one additional powdered substance selected from talc, mica, silica, polyamide and polymethylmethacrylate.

9. A cosmetic composition according to any preceding claim, **characterized in that** said fluorescent particles have a mean size by volume of 0.1 µm to 50 µm, and preferably 0.5 pm to 20 µm.

10. A method for making up and/or caring for keratinous substances, comprising applying thereto a cosmetic composition according to any preceding claim.

11. A method according to the preceding claim, **characterized in that** the keratinous substances are selected from the eyelashes, nails, skin, hair and lips.

## Patentansprüche

1. Kosmetische Zusammensetzung, enthaltend, in einem physiologisch verträglichen Medium, fluoreszierende Teilchen, umfassend Moleküle von mindestens einer im Inneren einer Matrix verkapselt fluoreszierenden organischen Verbindung, die mindestens zum Teil aus mindestens einem Metalloxid und aus Phosphor gebildet ist.

2. Kosmetische Zusammensetzung nach Anspruch 1, **dadurch gekennzeichnet, dass** die fluoreszierende organische Verbindung oder die fluoreszierenden organischen Verbindungen aus Fluoresceinen, Pyrazinen, Cumarinen, Naphthalimiden, Triazinen, Dioxazinen, Sulforhodaminen, Azo-Verbindungen, Azomethinen, Stilben-Derivaten, Oxazol-Derivaten, Benzoxazol, Imidiazol oder ihren Gemischen ausgewählt ist.

3. Kosmetische Zusammensetzung nach einem der Ansprüche 1 oder 2, **dadurch gekennzeichnet, dass** das oder die Metalloxide aus Oxiden von Silicium, Titan, Aluminium, Zirkonium oder ihren Gemischen ausgewählt ist.

4. Kosmetische Zusammensetzung nach einem der vorhergehenden Ansprüche, **dadurch gekennzeichnet, dass** das oder die Metalloxide aus Siliciumoxiden ausgewählt sind.

5. Kosmetische Zusammensetzung nach einem der vorhergehenden Ansprüche, **dadurch gekennzeichnet, dass** die flüssige Phase mindestens ein flüchtiges Lösungsmittel, insbesondere Wasser, einen Alkohol oder ein Öl enthält.

6. Kosmetische Zusammensetzung nach einem der vorhergehenden Ansprüche, **dadurch gekennzeichnet, dass** die fluoreszierenden Teilchen in einem Gehalt, gewichtsbezogen, von 0,5% bis 95%, und vorzugsweise von 1% bis 70%, und noch stärker bevorzugt von 5 bis 30%, bezogen auf das Gesamtgewicht der Zusammensetzung, vorhanden sind.

7. Kosmetische Zusammensetzung nach einem der vorhergehenden Ansprüche, **dadurch gekennzeichnet, dass** sie mindestens einen zusätzlichen, vorzugsweise streuenden Farbstoff, umfasst, der insbesondere aus Titandioxid oder Zinkoxid ausgewählt ist.

8. Kosmetische Zusammensetzung nach einem der vorhergehenden Ansprüche, **dadurch gekennzeichnet, dass** sie weiterhin mindestens einen pulverartigen Stoff umfasst, der insbesondere aus Talk, Mica, Siliciumdioxid, Polyamid, Polymethylmethacrylat ausgewählt ist.

9. Kosmetische Zusammensetzung nach einem der vorhergehenden Ansprüche, **dadurch gekennzeichnet, dass** die fluoreszierenden Teilchen eine mittlere Größe, volumenbezogen, von 0,1 µm bis 50 µm und vorzugsweise von 0,5 pm bis 20 µm aufweisen.

10. Schmink- und/oder Pflegeverfahren der Keratinsubstanzen bestehend aus dem Auftragen auf diese letzteren einer kosmetischen Zusammensetzung nach einem der vorhergehenden Ansprüche.

11. Verfahren nach einem der vorhergehenden Ansprüche, **dadurch gekennzeichnet, dass** die Keratinsubstanzen aus Wimpern, Nägeln, Haut, Haaren oder Lippen ausgewählt sind.
